(19) Europäisches Patentamt
European Patent Office
Office européen des brevets

(11) EP 0 852 945 B1

# (12) FASCICULE DE BREVET EUROPEEN

(45) Date de publication et mention de la délivrance du brevet:
**15.12.1999 Bulletin 1999/50**

(51) Int. Cl.$^6$: **A61K 7/48**, A61K 7/02

(21) Numéro de dépôt: **97402976.1**

(22) Date de dépôt: **09.12.1997**

(54) **Utilisation d'une composition gélifiée d'organopolysiloxane élastomérique associé à une phase grasse pour le démaquillage des yeux**

Gelierte Zusammensetzung eines Organopolysiloxans vom Elastomertyp in Kombination mit einer Fettphase zum Abschminken der Augen

Use of a gel composition containing an elastomeric organopolysiloxane and a fatty phase for eyes make up removal

(84) Etats contractants désignés:
**DE ES FR GB IT**

(30) Priorité: **24.12.1996 FR 9615982**

(43) Date de publication de la demande:
**15.07.1998 Bulletin 1998/29**

(73) Titulaire: **L'OREAL**
**75008 Paris (FR)**

(72) Inventeurs:
• **Vanstraceele-Magniere, Anne**
**21000 Dijon (FR)**
• **Marion, Catherine**
**92330 Sceaux (FR)**

(74) Mandataire:
**Tezier Herman, Béatrice**
**L' OREAL,**
**D.P.I.,**
**6, rue Bertrand Sincholle**
**92585 Clichy Cédex (FR)**

(56) Documents cités:
EP-A- 0 739 619      EP-A- 0 765 656
US-A- 5 525 344

• CHEMICAL ABSTRACTS, vol. 124, no. 4, 22 janvier 1996 Columbus, Ohio, US; abstract no. 37392, NAGANUMA, MASAYUKI ET AL: "Makeup cosmetics containing powdered organopolysiloxane elastomers, nonporous spherical silica, and oils" XP002044058 & JP 07 258 028 A (SHISEIDO CO LTD, JAPAN) 9 octobre 1995
• CHEMICAL ABSTRACTS, vol. 126, no. 8, 24 février 1997 Columbus, Ohio, US; abstract no. 108664, KURODA, AKIHIRO ET AL: "Sunscreens containing metal oxides, polyoxyalkylene-polysiloxanes, and elastomers or resin waxes" XP002044059 & JP 08 295 620 A (KANEBO LTD, JAPAN) 12 novembre 1996
• C.M. HANSEN: "The Three Dimensional Solubility Parameter - Key to Paint Component Affinites" JOURNAL OF PAINT TECHNOLOGY, vol. 39, no. 505, 1967, pages 104-113, XP002010040
• A. F. M. BARTON: CRC HANDBOOK OF SOLUBILITY PARAMETERS AND OTHER COHESION PARAMETERS, 1983, FLORIDA (USA), pages 153-161, XP002044057

**Description**

La présente invention se rapporte à une composition gélifiée par un organopolysiloxane pour le démaquillage des yeux, présentant une haute tolérance et permettant notamment le démaquillage des yeux sensibles. Elle s'applique aussi bien dans le domaine cosmétique que dermatologique.

Les compositions de démaquillage des yeux, classiquement utilisées, se présentent généralement sous forme de lotion ou de lait. En outre, elles contiennent des conservateurs en vue d'assurer une protection microbiologique et empêcher le développement de micro-organismes ; en particulier la pollution microbienne d'une composition est apportée par l'utilisateur, lors de la prise du produit avec les doigts. De plus, elles renferment des tensioactifs et des huiles permettant l'élimination du maquillage.

Ces compositions connues, bien que très efficaces, présentent un certains nombres d'inconvénients. En particulier, elles sont souvent difficiles à prélever et peuvent s'écouler entre les doigts. Par ailleurs, elles laissent une impression d'inconfort et de gêne, notamment de voile, sur les yeux et/ou les paupières. De plus, la présence de conservateurs et/ou de tensioactifs peut entraîner une irritation des yeux et/ou des paupières, pouvant provoquer des rougeurs, des démangeaisons, des picotements.

Le demandeur a découvert de manière surprenante que l'association d'un organopolysiloxane solide élastomérique au moins partiellement réticulé avec une phase grasse contenant une ou plusieurs huiles cosmétiques démaquillantes particulières, était susceptible d'éliminer efficacement le maquillage des yeux tout en remédiant notamment aux inconvénients ci-dessus. En particulier, les compositions contenant cette association ne procurent aucune gêne, ni voile, ni inconfort sur les yeux et/ou les paupières, après le démaquillage.

De façon plus précise, l'invention a pour objet une composition cosmétique ou dermatologique de démaquillage des yeux, contenant un milieu physiologiquement acceptable et gélifiée par au moins un organopolysiloxane solide élastomérique au moins partiellement réticulé associé à une phase grasse comprenant au moins une huile démaquillante choisie parmi l'isohexadécane et l'isododécane ou un mélange d'huiles comprenant au moins une huile démaquillante choisie parmi l'isohexadécane et l'isododécane, ledit mélange présentant des paramètres moyens de solubilité dD, dP et dH à 25° C de Hansen qui satisfont aux trois conditions suivantes :

(1) $dD \leq 20 \ (J/cm^3)^{\frac{1}{2}}$

(2) $dP \leq 10 \ (J/cm^3)^{\frac{1}{2}}$

(3) $dH \leq 15 \ (J/cm^3)^{\frac{1}{2}}$ .

On entend par 〈〈huile démaquillante〉〉, tout corps gras, liquide à température ambiante, physiologiquement acceptable et susceptible de dissoudre le ou les polymères, la ou les résines, la ou les cires, le ou les colorants et/ou le ou les plastifiants déposés sur les cils, les sourcils et/ou les paupières.

〈〈Par élastomérique〉〉, on entend un matériau souple, déformable ayant des propriétés viscoélastiques et notamment la consistance d'une éponge ou d'une sphère souple.

Par 〈〈physiologiquement acceptable〉〉, on entend compatible avec la peau, les yeux, les fibres kératiniques des êtres humains.

Certes, il est connu du document EP-A-295886 de Dow Corning des compositions de nettoyage de la peau contenant des particules d'organopolysiloxanes assurant une élimination des saletés et des cellules mortes de la peau par effet mécanique (scrub). Ces particules ne sont nullement utilisées pour former une composition gélifiée d'aspect homogène. En outre, la présence de ces particules de taille élevée (supérieure à 3μm) ne permet pas d'utiliser ces compositions pour le démaquillage des yeux et plus spécialement des yeux sensibles. Enfin, les compositions décrites dans ce document contiennent des tensioactifs, et ne sont donc pas appropriées pour le démaquillage des yeux sensibles.

Les compositions selon l'invention sont parfaitement adaptées aux yeux sensibles, c'est-à-dire aux personnes qui ressentent, aux niveaux des yeux, des brûlures, des échauffements, des picotements, des fourmillements, des inconforts et des tiraillements, résultant de l'emploi de certains cosmétiques, des variations de température rapides, des expositions aux rayonnements U.V., aux poussières ou aux courants d'air.

La définition des huiles dans l'espace de solubilité tridimensionnel selon Hansen est décrite dans l'article de C. M. Hansen : "The three dimensionnal solubility parameters" J. Paint Technol. 39, 105 (1967). Cet espace est défini par les paramètres dD, dP, dH ; ils sont exprimés en $(J/cm^3)^{\frac{1}{2}}$ :

- dD caractérise les forces de dispersion de London issues de la formation de dipôles induits lors des chocs moléculaires ;
- dP caractérise les forces d'interactions de Debye entre dipôles permanents ainsi que les forces d'interactions de Keesom entre dipôles induits et dipôles permanents ;

- dH caractérise les forces d'interactions spécifiques (type liaisons hydrogène, acide/base, donneur/accepteur, etc...).

Les organopolysiloxanes de la composition de l'invention présentent un remarquable pouvoir gélifiant d'huile. Ils ne sont pas desséchants pour la peau entourant les yeux et apportent de bonnes propriétés cosmétiques. Ces nouveaux élastomères conduisent à des compositions confortables à l'application, douces, non collantes et agréables au toucher. Cette douceur est due, d'une part à la texture des organopolysiloxanes et, d'autre part à leurs propriétés comparables à celles de microéponges piégeant les huiles notamment démaquillantes de la composition.

Les organopolysiloxanes de la composition selon l'invention sont partiellement ou totalement réticulés et de structure tridimensionnelle. Inclus dans une phase grasse telle que définie précédemment, ils se transforment, selon le taux de phase grasse utilisé, d'un produit d'aspect spongieux lorsqu'ils sont utilisés en présence de faibles teneurs en phase grasse en un gel homogène, en présence de quantités de phase grasse plus élevées.

Les compositions de l'invention sont sous forme d'un gel plus ou moins fluide ou d'une pâte d'aspect homogène. Ce type de présentation procure un certain nombre d'avantages comme la facilité de la prise du produit, sans perte significative, la facilité d'application et un dosage précis de la quantité de produit, nécessaire au démaquillage des yeux.

Les organopolysiloxanes de la composition selon l'invention peuvent être choisis parmi ceux décrits dans les documents EP-A-383 540 et EP-A-545 002 ou le brevet US-A-5 266 321. Selon ce brevet, ils sont choisis notamment parmi :

i) les organopolysiloxanes comprenant des motifs $R_2SiO$ et $RSiO_{1,5}$ et éventuellement des motifs $R_3SiO_{0,5}$ et/ou $SiO_2$ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs $R_2SiO$ sur les motifs $RSiO_{1,5}$ varie de 1/1 à 30/1 ;

ii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 % mol quand l'organopolysiloxane est non-cyclique et entre 1 et 50 % mol lorsque l'organopolysiloxane est cyclique.

Les organopolysiloxanes de la composition de l'invention sont par exemple ceux commercialisés sous les noms KSG6 par Shin-Etsu et Gransil par Grant Industries (SR-CYC, SR DMF10, SR-DC556), ou ceux commercialisés sous forme de gels déjà constitués (KSG15, KSG17, KSG16, KSG18 et KSG20 de Shin-Etsu, Gransil SR 5CYC gel, Gransil SR DMF 10 gel, Gransil SR DC556 gel de Grant Industries, SF 1204 et JK 113 de General Electric). On peut aussi utiliser un mélange de ces produits commerciaux.

Selon l'invention, les organopolysiloxanes élastomériques, lorsqu'ils se présentent sous forme de poudre, avant incorporation des huiles, ont une granulométrie au plus égale à 1 μm, pouvant descendre jusqu'à 0,5.

De façon préférentielle, le ou les organopolysiloxanes élastomériques sont présents dans la composition à une concentration, en matière active, allant de 0,1 à 30 % et plus préférentiellement de 3 à 25 % du poids total de la composition.

La phase grasse permettant le gonflement de l'organopolysiloxane élastomérique de l'invention comprend au moins une huile ou plusieurs huiles cosmétiques susceptibles de démaquiller les yeux, choisie(s) parmi l'isohexadécane ou l'isododécane, ou un mélange d'huiles comprenant ces huiles démaquillantes, ledit présentant des paramètres moyens de solubilité dD, dP et dH à 25°C selon l'espace de solubilité de Hansen qui satisfont aux conditions suivantes :

- (a) $dD \leq 20$ $(J/cm^3)^{1/2}$ et préférentiellement $10 \leq dD \leq 19$ $(J/cm^3)^{1/2}$

- (b) $dP \leq 10$ $(J/cm^3)^{1/2}$ et préférentiellement $dP \leq 7$ $(J/cm^3)^{1/2}$

- (c) $dH \leq 15$ $(J/cm^3)^{1/2}$ et préférentiellement $dH \leq 13$ $(J/cm^3)^{1/2}$ et plus préférentiellement $dH \leq 8$ $(J/cm^3)^{1/2}$ .

La phase grasse peut comprendre, outre l'isohexadécane et/ou l'isododécane, une ou plusieurs huiles démaquillantes répondant chacune aux conditions de solubilité définies ci-dessus ; ces huiles sont notamment choisies parmi :

- les hydrocarbures linéaires ou ramifiés, d'origine minérale ou synthétique comme l'huile de vaseline, le squalane et équivalents ;

- les esters d'acide gras à chaîne courte (inférieure à 12 atomes de carbone) tels que le néopentanoate d'octyldodécyle ;
- leurs mélanges.

Les compositions de l'invention présentent un haut pouvoir démaquillant, sans nécessiter de tensioactifs. Aussi, de façon avantageuse, elles sont exemptes de tensioactifs, ce qui leur confère une grande tolérance, et elles peuvent être utilisées plusieurs fois par jour si nécessaire, même par des personnes à yeux sensibles. En outre, elles permettent le démaquillage des compositions de maquillage des yeux (mascara, eye liner, crayon, fard, anti-cernes) résistant à l'eau, au sébum et/ou au transfert sur un support différent de la peau et des fibres kératiniques.

Par «exempt de tensioactif», il faut comprendre contenant moins de 1 % en poids de tensioactif, et mieux pas de tensioactif du tout.

De façon préférentielle, la phase grasse est présente dans la composition à une concentration allant de 5 à 90 % et plus préférentiellement de 20 à 80 % du poids total de la composition. Notamment du fait de ce taux élevé de phase grasse, il est possible d'obtenir une composition anhydre. Ce type de composition présente l'avantage, par rapport aux compositions contenant une grande quantité d'eau (supérieure à 10 %), de ne pas nécessiter de conservateurs. En effet, plus la quantité d'eau est élevée, plus le milieu est propice au développement des micro-organismes. Aussi, la composition de l'invention est avantageusement exempte de conservateurs, c'est-à-dire qu'elle contient moins de 1 % en poids de conservateur et mieux pas de conservateur du tout.

Avantageusement, les huiles démaquillantes participant au gonflement de l'organopolysiloxane représentent de 10 à 90 %, et mieux de 30 à 60 % du poids total de la composition.

La phase grasse conforme à l'invention peut contenir, en plus des huiles démaquillantes ci-dessus, une phase huileuse contenant des huiles classiquement utilisées dans le domaine cosmétique ou dermatologique, appelées huiles complémentaires, qui peuvent être démaquillantes ou non des yeux et qui, prises en tant que telles, ne répondent pas aux conditions de solubilité de Hansen définies ci-dessus. Conformément à l'invention, ces dernières doivent être mélangées avec des huiles démaquillantes appropriées telles que définies précédemment de manière à ce que le mélange total d'huiles (démaquillantes + non démaquillantes), satisfasse aux conditions de paramètres de solubilité de Hansen définies ci-dessus.

Comme huile démaquillante ne satisfaisant pas aux conditions ci-dessus, on peut citer notamment les esters d'acide gras à chaîne longue (supérieure à 12 atomes de carbone) comme le palmitate ou le myristate d'octyle ou d'isopropyle.

La phase huileuse peut donc contenir une ou plusieurs huiles complémentaires non démaquillantes choisies notamment parmi ;

- les huiles de silicones de faible viscosité (de préférence inférieure à 100 cst à 25° C) telles que les polysiloxanes linéaires ou ramifiés de faible degré de polymérisation comme méthylpolysiloxane, méthylphénylpolysiloxane, éthyl-méthylpolysiloxane, éthylphénylpolysiloxane, hydroxyméthylpolysiloxane, alkyl-polydiméthylsiloxane, et les polysiloxanes cycliques tels que octaméthyl-cyclopentasiloxane, décaméthylcyclopentasiloxane, ou leurs mélanges. Ces huiles de silicone ont l'avantage d'améliorer le toucher, de participer au gonflement de l'organopolysiloxane élastomérique et de satisfaire aux conditions de solubilité ci-dessus ;
- les huiles d'origine végétale comme les triglycérides liquides, par exemple les huiles de tournesol, de maïs, de soja, de courge, de pépins de raisin, de sésame, de noisette, d'abricot, de macadamia, de ricin, les triglycérides des acides caprylique/caprique comme ceux vendus sous les dénominations Miglyol 810, 812 et 818 par la société Dynamit Nobel.

Ces huiles non démaquillantes sont de préférence utilisées dans des concentrations allant de 0 à 50 % et plus préférentiellement de 0 à 40 % du poids total de la composition.

La composition gélifiée résultant de l'association des huiles ci-dessus et de l'organopolysiloxane peut être utilisée telle quelle et constituer elle-même une composition pour le démaquillage des yeux. Elle peut également être incorporée dans une formulation plus complexe de démaquillage des yeux en une quantité efficace pour obtenir à la fois la texture et la viscosité souhaitées et un bon démaquillage des yeux.

La composition selon l'invention peut, en outre, contenir des additifs classiquement utilisés dans le domaine concerné, ne présentant pas d'effets secondaires irritants ni d'intolérance avec les yeux tels que les agents hydratants et émollients. Ces additifs sont présents dans des quantités allant de préférence de 0 à 10 % du poids total de la composition.

L'invention se rapporte également à un procédé cosmétique de démaquillage des yeux, caractérisé en ce qu'on applique sur les yeux une quantité efficace d'une composition telle que définie précédemment.

Elle se rapporte aussi à l'utilisation d'au moins un organopolysiloxane élastomérique solide au moins partiellement réticulé, associé à une phase grasse comprenant au moins une huile démaquillante choisie parmi l'isohexadécane et l'isododécane ou un mélange d'huiles comprenant au moins une huile démaquillante choisie parmi l'isohexadécane et l'isododécane, ledit mélange présentant des paramètres moyens de solubilité dD, dP et dH à 25 °C de Hansen qui satisfont aux trois conditions suivantes :

(1) $dD \leq 20 \ (J/cm^3)^{\frac{1}{2}}$

(2) $dP \leq 10 \ (J/cm^3)^{\frac{1}{2}}$

(3) $dH \leq 15 \ (J/cm^3)^{\frac{1}{2}}$ ,

dans et/ou pour la préparation d'une composition cosmétique et/ou dermatologique pour démaquiller les yeux.

Les exemples qui suivent permettent d'illustrer l'invention. Les pourcentages sont donnés en poids.

**Exemple 1: Gel fluide démaquillant des yeux**

| | |
|---|---|
| - Mélange de 40% en poids huile de polydiméthylsiloxane 6 est et de 60% en poids de polydiméthylorganosiloxane partiellement réticulé vendu sous le nom KSG 6 par Shin Etsu | 25 % |
| - Isododécane | 50 % |
| - Cyclométhicone | 25 % |

On obtient un gel fluide facile à étaler, conférant aux yeux, après démaquillage et nettoyage avec un coton, un toucher doux de la peau entourant les yeux.

**Exemple 2: Gel démaquillant des yeux**

| | |
|---|---|
| - Mélange de 40% en poids huile de polydiméthylsiloxane 6 cst et de 60% en poids de polydiméthylorganosiloxane partiellement réticulé vendu sous le nom KSG 6 par Shin Etsu | 40 % |
| - Isododécane | 60 % |

On obtient un gel facile à étaler, conférant aux yeux, après démaquillage et nettoyage avec un coton, un toucher doux, sans voile.

L'efficacité et le confort des compositions 1 et 2 ci-dessus ont été évalués dans un premier test in vivo sur un panel de 10 personnes à peau et yeux sensibles, maquillées avec un mascara résistance à l'eau (waterproof en terminologie anglosaxonne).

Le pouvoir démaquillant et le confort se sont avérés particulièrement bons : la majorité des femmes a considéré le démaquillage tout à fait satisfaisant.

Un second test sur 46 personnes dont 72 % avaient les yeux sensibles avec la composition 2 a donné de très bons résultats. Ainsi, 82 % des femmes notent un bon, voire un très bon pouvoir démaquillant pour les yeux, supérieur (54 %) ou égal (20 %) à celui de leur produit habituel. Le confort oculaire est, hormis 2 cas de picotements et/ou de rougeur oculaire, satisfaisant pendant et après l'application.

En ce qui concerne l'aspect cosmétique, la texture et la teneur en matières grasses sont appréciées par 82 % des femmes et ce produit est à l'unanimité doux à l'application. Les paupières sont douces après démaquillage pour 80 % des femmes.

En conclusion, 76 % des femmes du panel se déclarent satisfaites de ce produit, principalement à cause de son bon pouvoir démaquillant.

**Revendications**

1. Utilisation pour le démaquillage des yeux d'une composition cosmétique gélifiée comprenant dans un milieu physiologiquement acceptable (a) au moins un organopolysiloxane solide élastomérique au moins partiellement réticulé et (b) une phase grasse comprenant au moins une huile démaquillante choisie parmi l'isohexadécane et l'isododécane, ou un mélange d'huiles comprenant au moins une huile démaquillante choisie parmi l'isohexadécane et l'isododécane, ledit mélange présentant des paramètres moyens de solubilité dD, dP et dH à 25 °C de Hansen qui satisfont aux trois conditions suivantes :

   (1) $dD \leq 20$ $(J/cm^3)^{1/2}$

   (2) $dP \leq 10$ $(J/cm^3)^{1/2}$

   (3) $dH \leq 15$ $(J/cm^3)^{1/2}$.

2. Utilisation selon la revendication 1, caractérisée en que la composition est sous forme de gel.

3. Utilisation selon la revendication 1 ou 2, caractérisée en que la composition est sous forme anhydre.

4. Utilisation selon l'une quelconque des revendications précédentes, caractérisée en ce que le milieu est exempt de tensioactif et/ou conservateur.

5. Utilisation selon l'une quelconque des revendications précédentes, caractérisée en ce que la phase grasse comprend au moins une huile démaquillante choisie parmi l'isohexadécane et l'isododécane, ou un mélange d'huiles comprenant au moins une huile démaquillante choisie parmi l'isohexadécane et l'isododécane, le mélange présentant des paramètres moyens de solubilité dD, dP et dH à 25°C de Hansen répondant aux trois conditions suivantes :

   (a) $10 \leq dD \leq 19$ $(J/cm^3)^{1/2}$

   (b) $dP \leq 7$ $(J/cm^3)^{1/2}$

   (c) $dH \leq 13$ $(J/cm^3)^{1/2}$.

6. Utilisation selon l'une quelconque des revendications précédentes, caractérisée en ce que l'huile démaquillante satisfaisante aux dites conditions, outre l'isohexadécane et/ou l'isododécane, est choisie parmi :

   - les hydrocarbures linéaires ou ramifiés, d'origine minérale ou synthétique ;
   - les esters d'acide gras à chaîne courte ;
   - leurs mélanges.

7. Utilisation selon l'une quelconque des revendications précédentes, caractérisée en ce que l'organopolysiloxane est choisi parmi :

   i) les polyorganopolysiloxanes comprenant des motifs $R_2SiO$ et $RSiO_{1,5}$ et éventuellement des motifs $R_3SiO_{0,5}$ et/ou $SiO_2$ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs $R_2SiO$ sur les motifs $RSiO_{1,5}$ varie de 1/1 à 30/1 ;
   ii) les organopolysiloxanes, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 % mole quand l'organopolysiloxane est non-cyclique et entre 1 et 50 % mole lorsque l'organopolysiloxane est cyclique.

8. Utilisation selon l'une quelconque des revendications précédentes, caractérisée en ce que la phase grasse contient, en outre, au moins une huile complémentaire démaquillante ou non ne satisfaisant pas aux dites conditions, mais dont le mélange avec l'huile démaquillante satisfaisant aux dites conditions, satisfait aux dites conditions.

9. Utilisation selon la revendication 8, caractérisée en ce que l'huile complémentaire est choisie parmi :

- les polysiloxanes linéaires ou ramifiés, les alkylpolydiméthylsiloxanes et les polysiloxanes cycliques ;
- les huiles d'origine végétale contenant des triglycérides ;
- les esters d'acide gras à chaîne longue ;
- leurs mélanges.

10. Utilisation selon l'une quelconque des revendications précédentes, caractérisée en ce que la phase grasse représente de 5 à 90 % du poids total de la composition.

11. Utilisation selon l'une quelconque des revendications précédentes, caractérisée en ce que l'organopolysiloxane est présent à une concentration en matière active allant de 0,1 à 30 % et de préférence de 3 à 25 % du poids total de la composition.

12. Utilisation selon l'une quelconque des revendications précédentes, caractérisée en ce que l'organopolysiloxane se présente sous forme de poudre, avant son introduction dans la composition, d'une granulométrie au plus égale à 1 $\mu$m.

13. Procédé de démaquillage cosmétique des yeux, caractérisé en ce que l'on applique sur les yeux une quantité efficace d'une composition gélifiée comprenant dans un milieu physiologiquement acceptable (a) au moins un organopolysiloxane solide élastomérique au moins partiellement réticulé et (b) une phase grasse comprenant au moins une huile démaquillante choisie parmi l'isohexadécane et l'isododécane, ou un mélange d'huiles comprenant au moins une huile démaquillante choisie parmi l'isohexadécane et l'isododécane, ledit mélange présentant des paramètres moyens de solubilité dD, dP et dH à 25 °C de Hansen qui satisfont aux trois conditions suivantes :

(1) $dD \leq 20 \ (J/cm^3)^{\frac{1}{2}}$

(2) $dP \leq 10 \ (J/cm^3)^{\frac{1}{2}}$

(3) $dH \leq 15 \ (J/cm^3)^{\frac{1}{2}}$.

## Claims

1. Use, in removing make-up from the eyes, of a gelled cosmetic composition comprising, in a physiologically acceptable medium, (a) at least one, at least partially crosslinked, solid elastomeric organopolysiloxane and (b) a fatty phase comprising at least one make-up removing oil chosen from isohexadecane and isododecane or a mixture of oils comprising at least one make-up removing oil chosen from isohexadecane and isododecane, the said mixture exhibiting mean Hansen solubility parameters dD, dP and dH at 25°C which satisfy the following three conditions:

(1) $dD \leq 20 \ (J/cm^3)^{\frac{1}{2}}$

(2) $dP \leq 10 \ (J/cm^3)^{\frac{1}{2}}$

(3) $dH \leq 15 \ (J/cm^3)^{\frac{1}{2}}$.

2. Use according to Claim 1, characterized in that the composition is in the form of a gel.

3. Use according to Claim 1 or 2, characterized in that the composition is in anhydrous form.

4. Use according to any one of the preceding claims, characterized in that the medium is devoid of surfactant and/or preservative.

5. Use according to any one of the preceding claims, characterized in that the fatty phase comprises at least one make-up removing oil chosen from isohexadecane and isododecane or a mixture of oils comprising at least one make-up removing oil chosen from isohexadecane and isododecane, the mixture exhibiting mean Hansen solubility parameters dD, dP and dH at 25°C corresponding to the following three conditions:

(a) $10 \leq dD \leq 19 \ (J/cm^3)^{\frac{1}{2}}$

(b) $dP \leq 7 \ (J/cm^3)^{\frac{1}{2}}$

(c) dH ≤ 13 $(J/cm^3)^{1/2}$.

6. Use according to any one of the preceding claims, characterized in that the make-up removing oil satisfying the said conditions, in addition to isohexadecane and/or isododecane, is chosen from:

- linear or branched hydrocarbons of mineral or synthetic origin;
- short-chain fatty acid esters;
- their mixtures.

7. Use according to any one of the preceding claims, characterized in that the organopolysiloxane is chosen from:

i) polyorganopolysiloxanes comprising $R_2SiO$ and $RSiO_{1.5}$ units and optionally $R_3SiO_{1.5}$ and/or $SiO_2$ units, in which the R radicals, independently of one another, denote a hydrogen, an alkyl, such as methyl, ethyl or propyl, an aryl, such as phenyl or tolyl, or an unsaturated aliphatic group, such as vinyl, and where the ratio by weight of the $R_2SiO$ units to the $RSiO_{1.5}$ units varies from 1/1 to 30/1;

ii) organopolysiloxanes, obtained by addition of an organohydropolysiloxane (1) to an organopolysiloxane (2) having unsaturated aliphatic groups, so that the amount of hydrogen or of unsaturated aliphatic groups in (1) and (2) respectively is between 1 and 20 mol% when the organopolysiloxane is noncyclic and between 1 and 50 mol% when the organopolysiloxane is cyclic.

8. Use according to any one of the preceding claims, characterized in that the fatty phase furthermore comprises at least one additional make-up removing oil or oil which is not make-up removing not satisfying the said conditions but which, as a mixture with the make-up removing oil satisfying the said conditions, does satisfy the said conditions.

9. Use according to Claim 8, characterized in that the additional oil is chosen from:

- linear or branched polysiloxanes, alkylpolydimethylsiloxanes and cyclic polysiloxanes;
- oils of vegetable origin comprising triglycerides;
- long-chain fatty acid esters;
- their mixtures.

10. Use according to any one of the preceding claims, characterized in that the fatty phase represents from 5 to 90% of the total weight of the composition.

11. Use according to any one of the preceding claims, characterized in that the organopolysiloxane is present at a concentration of active material ranging from 0.1 to 30% and preferably from 3 to 25% of the total weight of the composition.

12. Use according to any one of the preceding claims, characterized in that the organopolysiloxane is provided in the form of a powder, before it is introduced into the composition, with a particle size at most equal to 1 $\mu$m.

13. Process for cosmetically removing make-up from the eyes, characterized in that there is applied to the eyes an effective amount of a gelled composition comprising, in a physiologically acceptable medium, (a) at least one, at least partially crosslinked, solid elastomeric organopolysiloxane and (b) a fatty phase comprising at least one make-up removing oil chosen from isohexadecane and isododecane or a mixture of oils comprising at least one make-up removing oil chosen from isohexadecane and isododecane, the said mixture exhibiting mean Hansen solubility parameters dD, dP and dH at 25°C which satisfy the following three conditions:

(1) dD ≤ 20 $(J/cm^3)^{1/2}$

(2) dP ≤ 10 $(J/cm^3)^{1/2}$

(3) dH ≤ 15 $(J/cm^3)^{1/2}$.

# EP 0 852 945 B1

**Patentansprüche**

1. Verwendung einer gelierten kosmetischen Zusammensetzung zum Abschminken der Augen, die in einem physiologisch akzeptablen Medium (a) mindestens ein zumindest teilweise vernetztes festes Organopolysiloxan vom Elastomertyp und (b) eine Fettphase enthält, die mindestens ein reinigendes Öl, das unter Isohexadecan und Isododecan ausgewählt ist, oder ein Gemisch von Ölen, das mindestens ein reinigendes Öl aufweist, das unter Isohexadecan und Isododecan ausgewählt ist, enthält, wobei das Gemisch bei 25 °C mittlere Löslichkeitsparameter dD, dP und dH nach HANSEN aufweist, die die folgenden drei Bedingungen erfüllen:

   (1) $dD \leq 20 \ (J/cm^3)^{1/2}$

   (2) $dP \leq 10 \ (J/cm^3)^{1/2}$

   (3) $dH \leq 15 \ (J/cm^3)^{1/2}$ .

2. Verwendung nach Anspruch 1, dadurch gekennzeichnet, daß die Zusammensetzung in Gelform vorliegt.

3. Verwendung nach Anspruch 1 oder 2, dadurch gekennzeichnet, daß die Zusammensetzung in wasserfreier Form vorliegt.

4. Verwendung nach einem der vorhergehenden Ansprüche, dadurch gekennzeichnet, daß das Medium keinen grenzflächenaktiven Stoff und/oder kein Konservierungsmittel enthält.

5. Verwendung nach einem der vorhergehenden Ansprüche, dadurch gekennzeichnet, daß die Fetthase mindestens ein reinigendes Öl, das unter Isohexadecan und Isododecan ausgewählt ist, oder ein Gemisch von Ölen, das mindestens ein Öl aufweist, das unter Isohexadecan und Isododecan ausgewählt ist, enthält, wobei das Gemisch bei 25 °C mittlere Löslichkeitsparameter dD, dP und dH nach HANSEN aufweist, die den folgenden drei Bedingungen entsprechen:

   (a) $10 \leq dD \leq 19 \ (J/cm^3)^{1/2}$

   (b) $dP \leq 7 \ (J/cm^3)^{1/2}$

   (c) $dH \leq 13 \ (J/cm^3)^{1/2}$ .

6. Verwendung nach einem der vorhergehenden Ansprüche, dadurch gekennzeichnet, daß das reinigende Öl, das die genannten Bedingungen erfüllt, abgesehen von Isohexadecan und/oder Isododecan ausgewählt ist unter:

   - geradkettigen oder verzweigten Kohlenwasserstoffen mineralischen oder synthetischen Ursprungs,
   - Estern von kurzkettigen Fettsäuren und
   - den Gemischen dieser Verbindungen.

7. Verwendung nach einem der vorhergehenden Ansprüche, dadurch gekennzeichnet, daß das Organopolysiloxan ausgewählt ist unter:

   i) Organopolysiloxanen, die Einheiten $R_2SiO$ und $RSiO_{1,5}$ und gegebenenfalls Einheiten $R_3SiO_{0,5}$ und/oder $SiO_2$ aufweisen, worin die Gruppen R unabhängig voneinander Wasserstoff, eine Alkylgruppe, wie Methyl, Ethyl oder Propyl, eine Arylgruppe, wie Phenyl oder Tolyl, oder eine ungesättigte aliphatische Gruppe, wie Vinyl, bedeuten und worin das Gewichtsverhältnis der Einheiten $R_2SiO$ zu den Einheiten $RSiO_{1,5}$ im Bereich von 1/1 bis 30/1 liegt, und
   ii) Organopolysiloxanen, die durch Addition eines Organohydrogenopolysiloxans (1) und eines Organopolysiloxans (2) mit ungesättigten aliphatischen Gruppen so hergestellt werden, daß die Wasserstoffmenge oder die Menge ungesättigter aliphatischer Gruppen in (1) bzw. (2) im Bereich von 1 bis 20 Mol-% liegt, wenn das Organopolysiloxan nicht cyclisch vorliegt, und im Bereich von 1 bis 50 Mol-%, wenn das Organopolysiloxan cyclisch vorliegt.

8. Verwendung nach einem der vorhergehenden Ansprüche, dadurch gekennzeichnet, daß die Fettphase ferner mindestens ein ergänzendes reinigendes oder nicht reinigendes Öl enthält, das nicht den genannten Bedingungen entspricht, wobei jedoch das Gemisch mit dem reinigenden Öl, das die genannten Bedingungen erfüllt, den genannten Bedingungen entspricht.

9. Verwendung nach Anspruch 8, dadurch gekennzeichnet, daß das ergänzenden Öl ausgewählt ist unter:

- geradkettigen oder verzweigten Polysiloxanen, Alkylpolydimethylsiloxanen und cyclischen Polysiloxanen;
- Ölen pflanzlicher Herkunft, die Triglyceride enthalten;
- Estern von langkettigen Fettsäuren und
- den Gemischen dieser Verbindungen.

10. Verwendung nach einem der vorhergehenden Ansprüche, dadurch gekennzeichnet, daß die Fettphase 5 bis 90 % des Gesamtgewichts der Zusammensetzung ausmacht.

11. Verwendung nach einem der vorhergehenden Ansprüche, dadurch gekennzeichnet, daß das Organopolysiloxan in einer Wirkstoffkonzentration vorliegt, die im Bereich von 0,1 bis 30 % und vorzugsweise von 3 bis 25 % des Gesamtgewichts der Zusammensetzung liegt.

12. Verwendung nach einem der vorhergehenden Ansprüche, dadurch gekennzeichnet, daß das Organopolysiloxan bevor es in die Zusammensetzung eingebracht wird in Form eines Pulvers mit einer Korngröße von höchstens 1 $\mu$m vorliegt.

13. Kosmetisches Verfahren zum Abschminken der Augen, dadurch gekennzeichnet, daß auf die Augen eine wirksame Menge einer gelierten Zusammensetzung aufgebracht wird, die in einem physiologisch akzeptablen Medium (a) mindestens ein zumindest teilweise vernetztes festes Organopolysiloxan vom Elastomertyp und (b) eine Fettphase enthält, die mindestens ein reinigendes Öl, das unter Isohexadecan und Isododecan ausgewählt ist, oder ein Gemisch von Ölen, das mindestens ein reinigendes Öl aufweist, das unter Isohexadecan und Isododecan ausgewählt ist, enthält, wobei das Gemisch bei 25 °C mittlere Löslichkeitsparameter dD, dP und dH nach HANSEN aufweist, die die folgenden drei Bedingungen erfüllen:

(1) $dD \leq 20 \ (J/cm^3)^{1/2}$

(2) $dP \leq 10 \ (J/cm^3)^{1/2}$

(3) $dH \leq 15 \ (J/cm^3)^{1/2}$ .